# EUROPÄISCHE PATENTSCHRIFT

(11) **EP 2 792 388 B1**
(45) Veröffentlichungstag und Bekanntmachung des Hinweises auf die Patenterteilung: **20.09.2017**
(21) Anmeldenummer: 13002067.0
(22) Anmeldetag: 19.04.2013
(51) Int. Cl.: A61N 5/10

(54) **Bestrahlungseinrichtung**
Irradiation device
Dispositif de rayonnement

(43) Veröffentlichungstag der Anmeldung: 22.10.2014
(73) Patentinhaber: Scientific RT GmbH, 81373 München (DE)
(72) Erfinder: Sobotta, Benjamin, 72574 Bad Urach (DE); Alber, Markus, 70191 Stuttgart (DE)
(74) Vertreter: Spengler, Robert

(56) Entgegenhaltungen:
- WO-A1-2009/052845
- US-A1- 2010 303 205

## Beschreibung

Die vorliegende Erfindung betrifft eine Bestrahlungseinrichtung zum Bestrahlen eines Objektes aus wenigstens zwei relativ zum Objekt unterschiedlichen Einstrahlrichtungen, ein Computerprogrammprodukt mit Steueranweisungen zum Steuern einer Bestrahlungseinrichtung und ein Speichermedium, auf dem ein derartiges Computerprogrammprodukt gespeichert ist.

Bestrahlungseinrichtungen zum Bestrahlen eines Objektes aus wenigstens zwei relativ zum Objekt unterschiedlichen Einstrahlrichtungen werden unter anderem in der Strahlentherapie zur Behandlung von Krebserkrankungen eingesetzt und sind zum Beispiel aus der US 5,898,902, der US 7,961,843 B2 und der US 8,059,782 B2 bekannt. In der Strahlentherapie handelt es sich bei dem zu bestrahlenden Objekt um einen Patienten, der von der Bestrahlungseinrichtung aus mehreren unterschiedlichen Einstrahlrichtungen mit Röntgenstrahlung bestrahlt wird. Derartige bekannte Bestrahlungseinrichtungen weisen üblicherweise eine Strahlenquelle auf, die mit einer Mehrzahl von Blendenpaaren versehen ist, und die sich auf einer zumeist kreisförmigen Trajektorie um den Patienten bewegt, um dessen krankes Gewebe gemäß einem Behandlungsplan aus mehreren vorgegebenen Einstrahlrichtungen mit Röntgenstrahlung zu beaufschlagen, wobei mittels der Blendenpaare eine Querschnittsform eines von der Strahlenquelle emittierten Strahlungsfeldes einstellbar ist. Verfahren zum Erstellen von Behandlungsplänen sind beispielsweise in der US 7,880,154 B2 und der WO 2011/160235 A1 offenbart und ein Verfahren zur Optimierung von Behandlungsplänen beschreibt die US 8,238,520 B2.

Ein Grundproblem der Strahlentherapie besteht in den Nebenwirkungen, die sich bei der Bestrahlung von gesundem Gewebe ergeben und zu chronischen Langzeitschädigungen führen können. Da bei Patienten während der Zeitdauer der Bestrahlung natürliche Organbewegungen stattfinden, kann ein ungewolltes Bestrahlen gesunden Gewebes jedoch nie gänzlich ausgeschlossen werden Je kürzer der Bestrahlungsvorgang ist, umso geringer ist die Wahrscheinlichkeit, aufgrund der Organbewegungen ungewollt gesundes Gewebe zu bestrahlen. Zudem ist es insbesondere für betagte Patienten auch aus psychologischen und physiologischen Gründen von Vorteil, wenn deren Behandlung nicht allzu lange dauert Man ist aus diesen Gründen bestrebt, die Zeitdauer der Bestrahlung möglichst kurz zu halten.

Es ist daher die Aufgabe der vorliegenden Erfindung, eine Bestrahlungseinrichtung, ein Computerprogrammprodukt mit Steueranweisungen zum Steuern einer Bestrahlungseinrichtung und ein Speichermedium, auf dem ein derartiges Computerprogrammprodukt gespeichert ist, zu schaffen, mit denen bei einer vorgegebenen Dosisverteilung, die in einem zu bestrahlenden Objekt zu erzielen ist, eine Verkürzung der Bestrahlungszeit des Objektes erreicht werden kann.

Diese Aufgabe wird mit einer Bestrahlungseinrichtung mit den Merkmalen des Anspruchs 1, mit einem Computerprogrammprodukt gemäß dem Anspruch 9 und mit einem Speichermedium, nach Anspruch 10 gelöst. Bevorzugte Ausführungsformen der Erfindung sind Gegenstand der abhängigen Ansprüche.

Gemäß der vorliegenden Erfindung wird ausgehend von einer vorgegebenen Dosisverteilung, die angibt, welche Energie jeweiligen Volumenelementen des Objektes mit der Bestrahlung zuzuführen ist, wobei unter der Dosis die Energie pro Masse verstanden wird, und wobei die Dosis entweder direkt oder mittels einer Funktion, welche eine Präferenz für eine Dosis ausdrückt vorgegeben sein kann, unter Zuhilfenahme einer Kostenfunktion bzw Wichtungsfunktion bzw, Gewichtungsfunktion, welche einzelnen Kombinationen diskreter Positionen der Blendenelemente Kosten bzw. Wichtungen bzw. Gewichtungen entsprechend ihrer Zuträglichkeit zur zu erzielenden Dosisverteilung zuweist, eine Folge von jeweiligen sukzessiv aufeinanderfolgenden Kombinationen diskreter Positionen der beiden Blendenelemente für jeweilige sukzessiv aufeinanderfolgende Einstrahlrichtungen ermittelt. Dabei erfolgt die Auswahl nachfolgender Kombinationen diskreter Positionen für jeweilige nachfolgende Einstrahlrichtungen unter Berücksichtigung der Maximalgeschwindigkeit der Blendenelemente. Kombinationen diskreter Positionen für nachfolgende Einstrahlrichtungen, die aufgrund der begrenzten Maximalgeschwindigkeit der Blendenelemente in derjenigen Zeit, in welcher sich die Strahlenquelle von einer Stellung, in welcher deren Ausstrahlrichtung mit einer der Einstrahlrichtungen zusammenfällt zu einer Stellung bewegt, in welcher deren Ausstrahlrichtung mit der jeweils nachfolgenden Einstrahlrichtung zusammenfällt, nicht erreichbar sind, bleiben dabei von vorneherein unberücksichtigt.

Durch die Einbeziehung der Maximalgeschwindigkeit der Blendenelemente bei der Auswahl nächstfolgender Positionen ist gewährleistet, dass während der Zeit des Bewegens der Strahlenquelle von einer Stellung in die nächstfolgende Stellung die Blendenelemente die für diese nächstfolgende Stellung erforderlichen jeweiligen diskreten Positionen stets einnehmen können, also die Kombination diskreter Positionen der Blendenpaare für jede nächstfolgende Einstrahlrichtung stets gewährleistet ist, ohne dass die Strahlenquelle verlangsamt betrieben werden müsste, während die Auswahl auf der Grundlage der Kostenfunktion eine bestmögliche Annäherung der mit der Bestrahlung tatsächlich erzielten Dosisverteilung an die vorgegebene Dosisverteilung gewährleistet. Die Auswahl der nachfolgenden Kombination diskreter Positionen der Blendenelemente erfolgt somit letztendlich auf vorausschauende Weise unter simultaner Berücksichtigung mehrerer Gesamtenergieverläufe. Hierdurch wird es der Bestrahlungseinrichtung ermöglicht, die Strahlenquelle mit größerer Geschwindigkeit zu bewegen, wodurch sich die Gesamtdauer der Bestrahlung des Objektes wesentlich verkürzt. So ist es beispielsweise möglich, eine Prostatastrahlenbehandlung, bei welcher der Patient aus zwölf Einstrahlrichtungen, die jeweils um 30° versetzt sind, bestrahlt wird, innerhalb von 20 bis 25 Sekunden mit bis zu sechs Umläufen der Strahlenquelle und eine HNO-Strahlenbehandlung mit zehn Umläufen der Strahlenquelle und vier Sekunden pro Umlauf oder mit zwölf Umläufen der Strahlenquelle und fünf Sekunden pro Umlauf durchzuführen. Mit derzeit erhältlichen Bestrahlungseinrichtungen dauert eine Bestrahlung hingegen das Vier- bis Fünffache davon und bei älteren Bestrahlungseinrichtungen ist die Bestrahlungszeit sogar noch deutlich länger.

Obwohl bei der vorliegenden Erfindung diskrete Positionen der Blendenelemente betrachtet werden, bedeutet dies keinerlei Einschränkung für die Beweglichkeit der Blendenelemente der Strahlenquelle oder die Positionierbarkeit der vorderen Enden der Blendenelemente innerhalb der Durchgangsmaximalöffnung. Erfindungsgemäße Bestrahlungseinrichtungen können durchaus auch Bestrahfungseinrichtungen mit Strahlenquellen sein, deren Blendenelemente kontinuierlich entlang der Geraden bewegt werden können und bei denen die vorderen Enden der Blendenelemente auch außerhalb der diskreten Positionen beliebig innerhalb der Durchgangsmaximalöffnung positionierbar sind, solange sie nur die betrachteten diskreten Positionen einnehmen können.

Besonders bevorzugt handelt es sich bei dem Steuerverfahren um ein automatisiertes oder computerimplementiertes Steuerverfahren. Damit ist es möglich, Bestrahlungseinrichtungen automatisiert zu betreiben.

Ganz allgemein kann die Gerade, entlang der die Blendenelemente verschiebbar sind, einen beliebigen Winkel mit der Ausstrahlrichtung der emittierten Strahlung bilden, solange wenigstens ein Teil dieser emittierten Strahlung durch die Durchgangsmaximalöffnung treten kann. Für gewöhnlich bildet die Gerade mit der Ausstrahlrichtung aus Konstruktionsgründen einen rechten Winkel, bzw. sie ist normal oder orthogonal zur Ausstrahlrichtung.

Üblicherweise weist die Folge mehr sukzessiv aufeinanderfolgende Kombinationen diskreter Positionen auf als sukzessiv aufeinanderfolgende Einstrahlrichtungen, was bedeutet, dass die Strahlenquelle die den sukzessiv aufeinanderfolgenden Einstrahlrichtungen entsprechenden sukzessiven Stellungen mehrmals durchläuft. Hierzu wird die Strahlenquelle entlang einer Trajektorie bewegt, die bevorzugt kreisförmig ist, aber auch elliptisch oder schraubenförmig ausgebildet sein kann. Bei kreisförmigen und elliptisch ausgeformten Trajektorien kann die Strahlenquelle dieselben Stellungen mehrfach hintereinander einnehmen, je nachdem, wie oft und in welchem Ausmaß die kreisförmige oder elliptische Trajektorie von der Strahlenquelle durchlaufen wird, während sie im Falle einer schraubenförmigen Trajektorie sukzessive Stellungen in der Regel nur einmal einnimmt.

Besonders bevorzugt werden bei dem Steuerverfahren im Schritt e) für Kombinationen diskreter Positionen der Folge jeweilige nachfolgende Kombinationen diskreter Positionen der Folge unter zusätzlicher Berücksichtigung von Beschleunigungen der Blendenelemente ermittelt. Kombinationen diskreter Positionen, welche von den Blendenelementen nur dann erreichbar sind, wenn sie mit extremen oder physikalisch unmöglichen Beschleunigungen bewegt werden, können bei Berücksichtigung der Beschleunigung ausgeschlossen werden. Hierdurch kann die Anzahl potentiell geeigneter nachfolgender Kombinationen diskreter Positionen noch weiter reduziert werden. Zudem ist es bei Berücksichtigung der Beschleunigung möglich, Folgen sukzessiv aufeinanderfolgender Kombinationen diskreter Positionen der beiden Blendenelemente zu ermitteln, bei denen die einzelnen Blendenelemente während des Bestrahlens gemäßigte Bewegungsmuster ohne größere Beschleunigungen ausführen. Insgesamt kann die Strahlenquelle mittels einer derart ermittelten Folge mit der größtmöglichen Geschwindigkeit bewegt und die Gesamtdauer der Bestrahlung des Objektes auf den kleinstmöglichen Wert verkürzt werden, wodurch ein Betreiben der Bestrahlungseinrichtung mit ihren maximal möglichen Leistungswerten ermöglicht wird.

Wenn bei der Ermittlung der Folge sukzessiv aufeinanderfolgender Kombinationen diskreter Positionen die Beschleunigung berücksichtigt wird, müssen im Gegensatz zur Vorgehensweise ohne Berücksichtigung der Beschleunigung in der Regel mehrere Einstrahlrichtungen gleichzeitig betrachtet werden. So kann eine zunächst optimale Folge oder ein zunächst optimaler Teil einer Folge wie bereits erwähnt in eine Situation führen, in welcher die nächste Kombination diskreter Positionen aufgrund der nur endlichen Geschwindigkeitsänd e-rungen nicht erreicht werden kann. Daher werden auch Folgen oder Teilabschnitte von Folgen bei der Ermittlung der Folge im Schritt e) berücksichtigt, die zunächst nicht optimal erscheinen. Um den Aufwand der Ermittlung in Grenzen zu halten, sollten ungünstige Folgen oder Folgenabschnitte so früh wie möglich aussortiert werden, um die Anzahl der simultan verfolgten Folgen oder Folgenabschnitte möglichst gering zu halten. Da sich der Einfluss der Beschleunigung auf lediglich drei aufeinanderfolgende Einstrahlrichtungen auswirkt, ist es möglich, suboptimale Folgen oder Folgenabschnitte relativ schnell auszuschließen.

Bei einer bevorzugten Ausführungsform werden im Schritt e) die nachfolgenden Kombinationen diskreter Positionen der Folge unter Berücksichtigung der Maximalgeschwindigkeit der Blendenelemente in einem ersten Schritt vorausgewählt und in einem zweiten Schritt wird von den im ersten Schritt vorausgewählten Kombinationen diskreter Positionen wenigstens eine Kombination diskreter Positionen in Abhängigkeit von den ihr zugewiesenen Kosten ausgewählt. Die den Kombinationen diskreter Positionen der beiden Blendenelemente entsprechend der Kostenfunktion zugewiesenen Kosten können umso größer sein, je größer der Beitrag der jeweiligen Kombination zum jeweiligen Gesamtenergieverlauf ist, oder sie können umso kleiner sein, je größer der Beitrag der jeweiligen Kombination zum zugehörigen Gesamtenergieverlauf ist. Im ersten Fall wird bei der im zweiten Schritt erfolgenden Auswahl von Kombinationen aus der im ersten Schritt erfolgten Vorauswahl von Kombinationen vorteilhaft diejenige Kombination mit den höchsten zugewiesenen Kosten ausgewählt, während im zweiten Fall diejenige Kombination mit den niedrigsten zugewiesenen Kosten ausgewählt wird. Allgemein kann aus der Vorauswahl eine Kombination diskreter Positionen ausgewählt werden, deren Kosten im Vergleich zu den Kosten der anderen Kombinationen dieser Vorauswahl ein Extremum bilden. Andere Ausführungsformen der vorliegenden Erfindung führen zuerst eine Vorauswahl von Kombinationen diskreter Positionen anhand der ihnen zugewiesenen Kosten aus und wählen anschließend aus diesen vorausgewählten Kombinationen diskreter Positionen diejenigen aus, die unter Berücksichtigung der Maximalgeschwindigkeit überhaupt erreichbar sind. Dabei kann die Ermittlung der Folge auch in mehreren alternativen Durchläufen erfolgen, bei denen mehrere alternative Folgen ermittelt werden, die miteinander verglichen werden um letztendlich diejenigen im Schritt e) ausgewählten Kombinationen auszuwählen, die in den einzelnen Folgen am häufigsten vorkommen, oder um von Folge zu Folge die am wenigsten vorkommenden Kombinationen auszusortieren, bis nur eine Kombination für jeweilige Einstrahlrichtungen übrigbleibt.

Da die meisten Bestrahlungseinrichtungen nicht nur ein Blendenpaar sondern eine Mehrzahl von Blendenpaaren aufweisen, ist es ferner besonders bevorzugt, wenn mit dem Steuerverfahren eine Bestrahlungseinrichtung gesteuert wird, die eine Strahlenquelle mit einer Mehrzahl von Blendenpaaren aufweist, und bei dem die Schritte c) bis f) für jedes der Blendenpaare durchgeführt werden. Vorteilhafterweise weist die Bestrahlungseinrichtung dabei eine Mehrzahl von interdigitierend angeordneten Blendenpaaren auf, d.h. dass die Blendenelemente der jeweiligen Blendenpaare nebeneinander angeordnet und entlang jeweiliger paralleler Geraden verschiebbar sind, ohne dabei Blendenelemente benachbart angeordneter Blendenpaare zu beeinträchtigen.

Die im Schritt e) des Steuerverfahrens ermittelte Folge stellt im Falle einer strahlentherapeutischen Bestrahlungseinrichtung den Behandlungsplan dar, gemäß dem ein Patient, der das zu bestrahlende Objekt bildet, zu bestrahlen ist. Bevor die Bestrahlung des Patienten stattfindet, wird bei strahlentherapeutischen Bestrahlungseinrichtungen der Bestrahlungsplan zunächst von einer Datenverarbeitungsvorrichtung oder einem Computer oder einem Prozessor ermittelt, die oder der zumeist nicht Teil der Bestrahlungsvorrichtung ist. Daher wird wenigstens einer der Schritte c) bis e) des Steuerverfahrens bevorzugt von einer Datenverarbeitungsvorrichtung ausgeführt. Die Datenverarbeitungsvorrichtung kann dabei für die Ermittlung der Folge bekannte Suchalgorithmen verwenden, wie zum Beispiel einen Suchalgorithmus entsprechend dem "kürzeste-Pfad-Problem der Graphentheorie" oder dem "kürzeste-Pfad-Problem der Graphentheorie mit Nebenbedingungen", welcher vorteilhafterweise bei der Ermittlung der Folge im Schritt e) mit Berücksichtigung der Beschleunigung verwendet wird.

Bei gewissen Ausführungsformen der vorliegenden Erfindung werden die Schritte a) bis e) nicht nur einmal, sondern mehrfach ausgeführt. So ist es möglich, den Schritt a) wenigstens zweimal oder mehrfach auszuführen oder die Schritte a) bis e) wenigstens zweimal oder mehrfach auszuführen. Hierdurch kann die Bestrahlung des Objektes optimiert werden. Beispielsweise kann die Dosisverteilung für jeden Umlauf der Strahlenquelle auf bekannte Weise neu bestimmt und vorgegeben bzw. direkt oder über eine Funktion, welche eine Präferenz für eine Dosis ausdrückt, vorgegeben werden, um die Gesamtenergieverläufe für jeden Umlauf der Strahlenquelle auf der Grundlage der jeweiligen neuen Dosisverteilung, neu zu bestimmen. Andererseits kann die Dosisverteilung anstatt für alle Umläufe der Strahlenquelle nur für ausgesuchte Umläufe neu bestimmt und neu vorgegeben werden, beispielsweise für jeden zweiten oder fünften Umlauf.

Vorteilhafterweise wird von der Strahlenquelle während des Bewegens von wenigstens einer der Stellungen zur jeweiligen nächstfolgenden Stellung kontinuierlich Strahlung emittiert und/oder die Strahlenquelle ist während des Bewegens von wenigstens einer der Stellungen zur jeweiligen nächstfolgenden Stellung deaktiviert. Beispielsweise ist es zu Beginn eines Bestrahlungsprozesses oftmals von Vorteil, wenn die Strahlenquelle auf gewissen Bewegungsabschnitten beim Wechsel zwischen den Stellungen aktiviert bleibt und kontinuierlich Strahlung emittiert, während es gegen Ende des Bestrahlungsprozesses, wenn bereits eine gewisse Energiemenge in das Objekt eingebracht worden ist, eher vorteilhaft sein kann, die Strahlungsquelle während ihrer Bewegung stroboskopartig zu betreiben, indem sie abwechselnd immer wieder deaktiviert und aktiviert wird. Sofern die Strahlenquelle beim Bewegen zwischen den Stellungen für den Bestrahtungsprozess nicht wechselweise aktiviert und deaktiviert werden muss, ist das Steuerverfahren auch für Bestrahlungseinrichtungen mit Strahlenquellen anwendbar, bei denen ein ausreichend schnelles Ein- und Abschalten der Strahlenquelle nicht möglich ist. Hierdurch kann die erfindungsgemäße Bestrahlungseinrichtung vereinfacht ausgeführt sein, da auf die notwendigen Einrichtungen zum ausreichend schnellen Ein- und Abschalten der Strahlenquelle verzichtet werden kann.

Die vorliegende Erfindung wird nachfolgend anhand eines bevorzugten Ausführungsbeispiels unter Zuhilfenahme von Figuren näher erläutert. Dabei zeigen.
- Figur 1: einen prinzipiellen Aufbau einer Strahlenquelle einer Bestrahlungseinrichtung in stark vereinfachter Form;
- Figur 2: diskrete Positionen von Blendenelementen eines Blendenpaares;
- Figur 3: eine Prinzipskizze, welche die Bewegung einer Strahlenquelle um einen Patienten zeigt;
- Figur 4: ein Flussdiagramm für ein Steuerverfahren zum Steuern einer Bestrahlungseinrichtung;
- Figur 5: einen Gesamtenergieverlauf mit zugehöriger Kostenfunktion;
- Figur 6: eine Auswahl sukzessiver diskreter Positionen eines Blendenelementes in symbolisierter Darstellung.

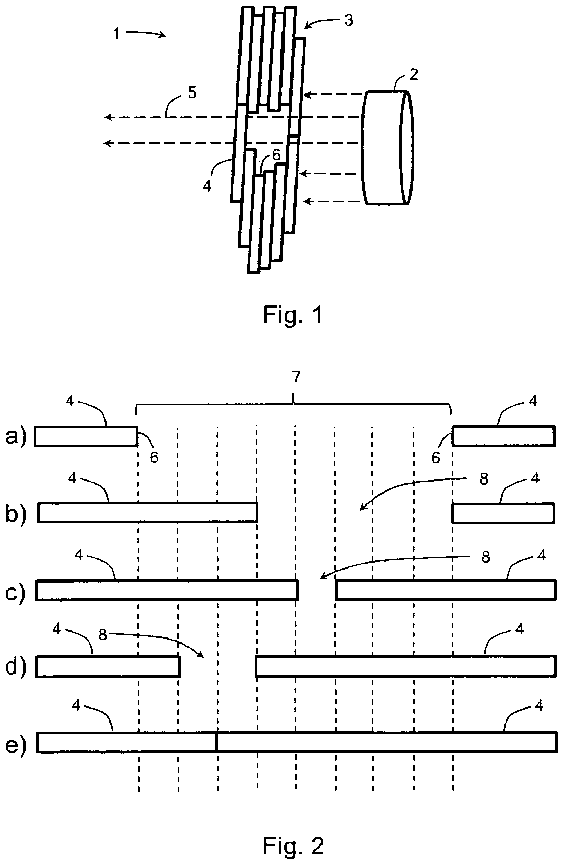

Ein Steuerungsverfahren zum Steuern einer erfindungsgemäßen Bestrahlungseinrichtung wird im Folgenden am Beispiel einer strahlentherapeutischen Bestrahlungseinrichtung mit einer Strahlenquelle (1) eines an sich bekannten Aufbaus erläutert. In der Figur 1 ist der prinzipielle Aufbau der Strahlenquelle (1) stark vereinfacht dargestellt.

Die Strahlenquelle (1) umfasst im Wesentlichen ein Strahlungserzeugungsmittel (2) zum Erzeugen von Röntgenstrahlung, dies sich für klinische Anwendungen typischerweise im Bereich zwischen 6 MeV und 15 MeV befindet, sowie eine Mehrzahl von aneinander anliegenden Blendenpaaren (3) mit jeweils zwei aneinander gegenüberliegenden Blendenelementen (4). Obwohl in der Figur 1 aus darstellerischen Gründen nur einige wenige Blendenpaare (3) zu sehen sind, weist die Strahlenquelle (1) tatsächlich typischerweise zwischen 40 bis 80 derartiger Blendenpaare (3) auf. Vom Strahlungserzeugungsmittel (2) mit einem vorgegebenen Intensitätsprofil erzeugte Röntgenstrahlung wird von diesem in einer Ausstrahlrichtung (5) emittiert.

Bei den Blendenelementen (4) handelt es sich im vorliegenden Fall um 8 cm lange blattförmige Wolframkörper, die für die von dem Strahlungserzeugungsmittel (2) emittierte Röntgenstrahlung nahezu undurchdringlich und mit einer 3-10 mm breiten Seitenfläche senkrecht zur Ausstrahlrichtung (5) angeordnet sind. Je zwei Blendenelemente (4) eines jeden Blendenpaares (3) sind mit einander zugewandten Seitenflächen oder Enden (6) angeordnet und entlang einer zur Ausstrahlrichtung (5) senkrechten Geraden verschiebbar. Alle Geraden, entlang denen die Blendenelemente (4) der jeweiligen Blendenpaare (3) verschiebbar sind, sind zueinander parallel ausgerichtet, so dass Blendenelemente (4) benachbarter Blendenpaare (3) beliebig verschoben werden können, ohne sich gegenseitig zu behindern; in anderen Worten sind die Blendenpaare (3) interdigitierend angeordnet. Entlang der jeweiligen Geraden können die Blendenelemente (4) der Blendenpaare (3) mehrere diskrete Positionen einnehmen. Dies ist in der Figur 2 anhand eines der Blendenpaare (3) beispielhaft für alle anderen Blendenpaare (3) der Strahlenquelle (1) dargestellt.

Im in der Figur 2 gezeigten Fall a) nehmen die Blendenelemente (4) Positionen ein, in denen ihre Enden (6) am weitesten voneinander beabstandet sind. In diesen Positionen der Blendenelemente (4) bilden deren Enden (6) einander gegenüberliegende Begrenzungen einer länglichen Durchgangsmaximalöffnung (7) größtmöglicher Breite für die Strahlung, die von dem Strahlungserzeugungsmittel (2) in der zur Breite der Durchgangsmaximalöffnung (7) senkrechten Ausstrahlrichtung (5) emittiert wird. Außer den Positionen des Falles a) können die Blendenelemente (4) nach Verschieben entlang der zur Ausstrahlrichtung (5) senkrechten und zur Durchgangsmaximalöffnung (7) parallelen Geraden weitere diskrete Positionen einnehmen, in denen der Abstand zwischen ihren einander gegenüberliegenden Enden (6) kleiner ist als im Falle a). In der Figur 2 zeigen gestrichelte Linien die Orte an, an welchen sich die Enden (6) der Blendenelemente (4) befinden, wenn die Blendenelemente (4) die verschiedenen diskreten Positionen einnehmen. Dabei werden die von einem Blendenelement (4) erreichbaren diskreten Positionen durch die vom jeweils anderen Blendenelement (4) eingenommene Position eingeschränkt. Nimmt beispielsweise das in der Figur 2 linke Blendenelement (4) wie im Falle a) die äußerste linke Position ein, so ist es dem in der Figur 2 rechten Blendenelement (4) möglich, alle diskreten Positionen einzunehmen und sein dem linken Blendenelement (4) zugewandtes Ende (6) an jeden durch eine gestrichelte Linie gekennzeichneten Ort zu bringen. Dasselbe gilt umgekehrt. Wird das in der Figur 2 linke Blendenelement (4) jedoch nach rechts verschoben und sein dem rechten Blendenelement (4) zugewandtes Ende (6) dadurch an einen durch eine gestrichelte Linie gekennzeichneten Ort verschoben, der weiter rechts liegt als im Fall a), so sind diejenigen diskreten Positionen für das rechte Blendenelement (4) nicht mehr erreichbar, an denen sich sein dem linken Blendenelement (4) zugewandtes Ende (6) an einem Ort befindet, der sich weiter links befindet als das dem rechten Blendenelement (4) zugewandte Ende des linken Blendenelementes (4). Sofern nur eines der Blendenelemente (4) eine andere diskrete Position einnimmt als im Falle a), sind die einander zugewandten Enden (6) der Blendenelemente (4) einander näher als bei der Durchgangsmaximalöffnung (7) des Falles a). In allen diesen Fällen, von denen die Fälle b), c) und d) in der Figur 2 lediglich eine exemplarische Auswahl bilden, wird von den einander zugewandten Enden (6) der Blendenelemente (4) eine jeweilige Durchgangsöffnung (8) begrenzt, bei der es sich um einen Teilabschnitt der Durchgangsmaximalöffnung (7) handelt. Je nachdem, welche diskreten Positionen von den Blendenelementen (4) nun eingenommen werden, variieren die Breite und die Lage der jeweiligen Durchgangsöffnung (8). Insbesondere kann die Durchgangsöffnung (8) auch verschwinden, wenn nämlich die Blendenelemente (4) wie im Fall e) der Figur 2 diskrete Positionen einnehmen, bei denen deren einander zugewandten Enden (6) aneinander anliegen, so dass die in der Ausstrahlrichtung (5) emittierte Strahlung über die gesamte Breite der Durchgangsmaximalöffnung (7) vollständig abgeblockt wird. Es können aber auch Blendenpaare (3) vorgesehen sein, deren Blendenelementen (4) es nicht möglich ist, aneinander anzuliegen und die stets einen Durchgangsöffnung (8) begrenzen. Dabei werden unter einer Kombination diskreter Positionen der Blendenelemente (4) eines Blendenpaares (3) zwei diskrete Positionen verstanden, die von den Blendenelementen (4) eingenommen werden oder einzunehmen sind, d.h. die Kombination diskreter Positionen stellt ein 2-Tupel aus der Menge aller möglichen diskreten Positionen für die Blendenelemente (4) dar.

Da die Blendenelemente (4) aller Blendenpaare (3) wie im Zusammenhang mit der Figur 2 beschrieben eine Mehrzahl diskreter Positionen einnehmen können und da die Blendenpaare (3) interdigitierend angeordnet sind, lassen sich durch entsprechendes Positionieren der Blendenelemente (4) der einzelnen Blendenpaare (3) unterschiedliche Querschnittsformen für die von der Strahlenquelle (1) emittierte Röntgenstrahlung einstellen.

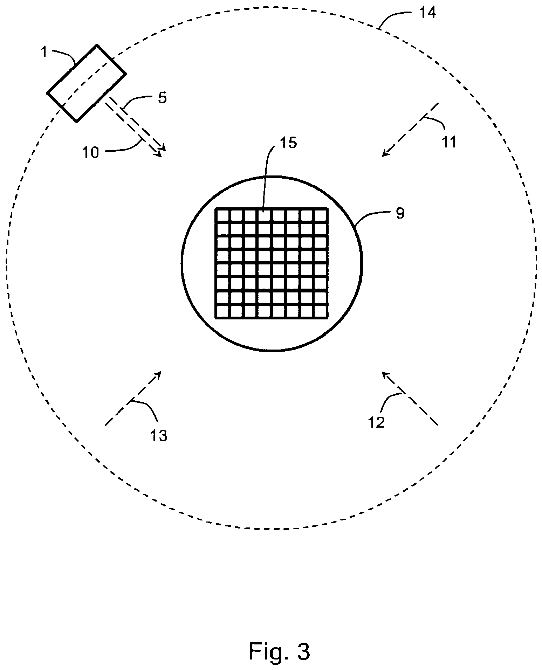

In der Strahlentherapie wird die Bestrahlungseinrichtung mit der beschriebenen Strahlenquelle (1) wie in der Prinzipskizze der Figur 3 gezeigt zum Bestrahlen eines Patienten (9) als dem zu bestrahlenden Objekt aus verschiedenen Einstrahlrichtungen (10, 11, 12, 13) eingesetzt, wobei in der Figur 3 aus Gründen der besseren Übersichtlichkeit lediglich vier Einstrahlrichtungen (10, 11, 12, 13) zu sehen sind, obwohl in der Praxis zumeist wesentlich mehr Einstrahlrichtungen vorgesehen werden. Hierzu kann sich die Strahlenquelle (1) entlang einer im vorliegenden Fall kreisförmigen Trajektorie (14) bewegen und jeweilige verschiedene Stellungen einnehmen, in denen ihre Ausstrahlrichtung (5) mit einer jeweiligen der Einstrahlrichtungen (10, 11, 12, 13) zusammenfällt. In der Situation der Figur 3 ist die Strahlenquelle (1) in einer Stellung zu sehen, in welcher ihre Ausstrahlrichtung (5) mit der Einstrahlrichtung (10) zusammenfällt. Bewegt sich die Strahlenquelle (1) im Uhrzeigersinn entlang der Trajektorie (14) um 90°, so gelangt sie in eine Stellung, in welcher ihre Ausstrahlrichtung (5) mit der Einstrahlrichtung (11) zusammenfällt. Nach Bewegen im Uhrzeigersinn um weitere 90° entlang der Trajektorie (14) gelangt sie in eine Stellung, in welcher ihre Ausstrahlrichtung (5) mit der Einstrahlrichtung (12) zusammenfällt und nach abermaligem Bewegen um 90° entlang der Trajektorie (14) gelangt sie in eine Stellung, in welcher ihre Ausstrahlrichtung (5) mit der Einstrahlrichtung (13) zusammenfällt. Schließlich gelangt die Strahlenquelle (1) nach weiteren 90° wieder in die in der Figur 3 gezeigte ursprüngliche Stellung. Die Strahlenquelle (1) kann den Patienten (9) beliebig oft entlang der Trajektorie (14) umkreisen.

Ziel der Bestrahlung des Patienten (9) ist es, mittels der Strahlung eine vorgegebene Energiemenge in bestimmte Volumenelemente (15) des Körpers des Patienten (9) einzubringen, wobei die einzubringende Energiemenge gewöhnlich nicht für alle Volumenelemente (15) gleich ist. Mit einer Dosisverteilung lässt sich die in ein Volumenelement (15) einzubringende Energiemenge in Abhängigkeit von der räumlichen Position und Größe des Volumenelementes (15) beschreiben. Wie Dosisverteilungen gewonnen werden ist dem Fachmann hinlänglich bekannt und soll daher an dieser Stelle auch nicht weiter ausgeführt werden.

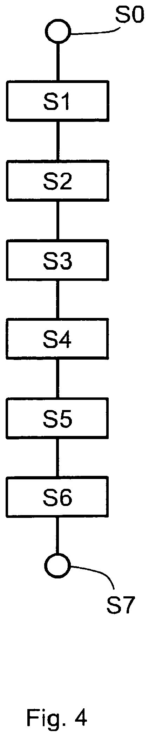

In der Figur 4 ist ein Flussdiagramm eines Steuerverfahrens für die Bestrahlungseinrichtung mit der Strahlenquelle (1) gezeigt. Das Verfahren beginnt mit dem Schritt S0 Im nachfolgenden Schritt S1 wird auf bekannte Weise eine zu erzielende Dosisverteilung vorgegeben, welche die jeweiligen Volumenelementen (15) des Körpers des Patienten (9) von der Strahlung zuzuführende Energie beschreibt. Sodann werden im Schritt S2 die Einstrahlrichtungen (10, 11, 12, 13) vorgegeben, aus denen der Patient (9) sukzessiv aufeinanderfolgend zu bestrahlen ist.

Um die gegebene Dosisverteilung im Körper des Patienten (9) zu erzielen, muss der Patient (9) aus den vorgegebenen Einstrahlrichtungen (10, 11, 12, 13) jedoch auf unterschiedliche Weise bestrahlt werden. Dabei sind in der Regel nicht nur die Querschnittsformen der auf den Patienten (9) aus den verschiedenen Einstrahlrichtungen (10, 11, 12, 13) einfallenden Röntgenstrahlungen für jede der Einstrahlrichtungen (10, 11, 12, 13) verschieden, sondern auch die Beträge der von der Röntgenstrahlung transportierten Gesamtenergie variieren über der Querschnittsfläche der emittierten Strahlung. So kann es Bereiche in der Querschnittsfläche der Strahlung geben, durch die mehr Gesamtenergie von der Röntgenstrahlung transportiert werden muss als in anderen Bereichen der Querschnittsfläche, wobei diese Bereiche für jede der Einstrahlrichtungen (10, 11, 12, 13) verschieden ausgebildet sein können. Da das Strahlungserzeugungsmittel (2) Röntgenstrahlung mit einem vorgegebenen Intensitätsprofil emittiert, müssen diese Verteilungen der von der Röntgenstrahlung transportierten Gesamtenergie über die Querschnittsfläche der emittierten Strahlung für jede der Einstrahlrichtungen (10, 11, 12, 13) durch geeignetes Positionieren der Blendenelemente (4) erzeugt werden, indem man beispielsweise sukzessive verschieden breite und verschieden angeordnete Durchgangsöffnungen (8) durch geeignete Kombinationen diskreter Positionen der Blendenelemente (4) bildet und deren Breite und Lage dabei so wählt, dass bei Durchgang der emittierten Strahlung durch die sukzessiv gebildeten Durchgangsöffnungen (8) eine gewünschte Verteilung der von der Röntgenstrahlung transportierten Gesamtenergie über die Querschnittsfläche der emittierten Strahlung erreicht wird.

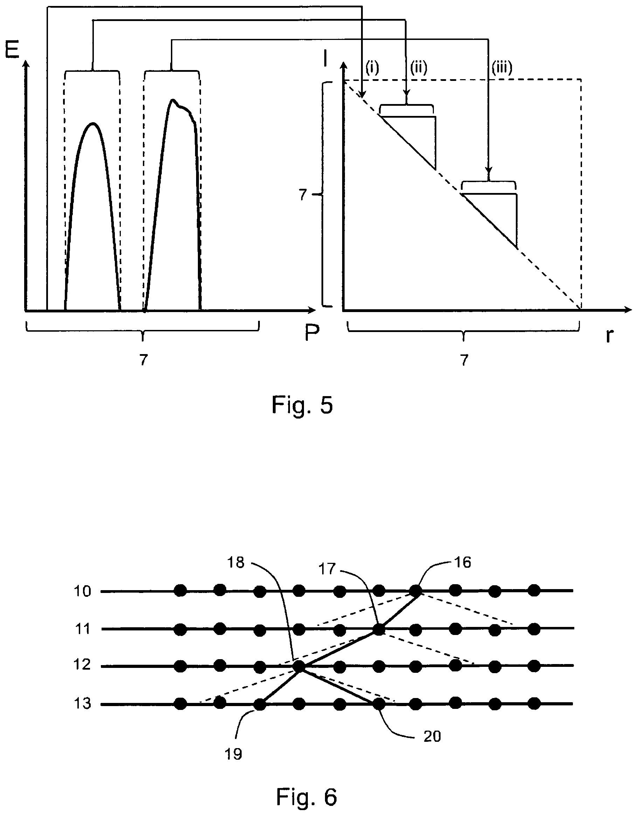

In der Figur 5 ist hierzu im linken Diagramm ein beispielhafter Gesamtenergieverlauf für die Einstrahlrichtung (10) mit zwei separaten Energiespitzen aufgezeichnet, der für Punkte in der Durchgangsmaximalöffnung (7) eines einzigen der Blendenpaare (3), für das mögliche diskrete Positionen P der Blendenelemente (4) entlang der Abszisse aufgetragen sind, an der Ordinate die jeweilige Gesamtenergie E angibt, welche von der Strahlung durch diese Punkte transportiert werden muss, damit sich mit entsprechenden Gesamtenergieverläufen für die übrigen Blendenpaare (3) die Verteilung der von der Röntgenstrahlung zu transportierenden Gesamtenergie über die Querschnittsfläche der emittierten Strahlung für die Einstrahlrichtung (10) ergibt, so dass nach Bestrahlen des Patienten (9) aus allen Einstrahlrichtungen (10, 11, 12, 13) entsprechend den jeweiligen Gesamtenergieverläufen die vorgegebene Dosisverteilung in dessen Körper erzielt wird. Dieser Gesamtenergieverlauf wird im Schritt S3 des in der Figur 4 gezeigten Flussdiagrammes insbesondere aus der Dosisverteilung und der Intensität bzw. dem vorgegebenen Intensitätsprofil der emittierten Strahlung abgeleitet.

Auf der Grundlage derartiger Gesamtenergieverläufe werden sodann im Schritt S4 Kostenfunktionen für jede der Einstrahlrichtungen (10, 11, 12, 13) erstellt, welche jeder Kombination diskreter Positionen der beiden Blendenelemente (4) eines Blendenpaares (3) jeweilige Kosten entsprechend dem Beitrag zuweisen, den diejenige Energie zum Gesamtenergieverlauf des betreffenden Blendenpaares (3) leistet, die von Röntgenstrahlung transportiert wird, welche in einer vorgegebenen Zeitdauer durch die jeweilige Durchgangsöffnung (8) tritt, die gebildet wird, wenn die Blendenelemente (4) diskrete Positionen entsprechend der Kombination diskreter Positionen einnehmen.

Ein vereinfachtes Beispiel für eine derartige Kostenfunktion ist im rechten Diagramm der Figur 5 zu sehen, auf dessen mit r bezeichneter Achse diskrete Positionen des rechten Blendenelementes (4) der Figur 2 aufgetragen sind und auf dessen mit I bezeichneter Achse diskrete Positionen des linken Blendenelementes (4) der Figur 2 aufgetragen sind. Besagte Kostenfunktion weist nun Kombinationen diskreter Positionen die Kosten 0 zu, wenn sie keinen Beitrag zum Gesamtenergieverlauf des linken Diagramms der Figur 5 leisten, und sie weist Kombinationen diskreter Positionen die Kosten 1 zu, wenn sie zu diesem Gesamtenergieverlauf beitragen. Da alle möglichen diskreten Positionen des linken und rechten Blendenelementes (4) auf der Abszisse und der Ordinate aufgetragen sind, sind alle möglichen Kombinationen diskreter Positionen dieser beiden Blendenelemente (4) innerhalb eines Quadrates eindeutig identifizierbar, dessen Seitenlänge der Breite der Durchgangsmaximalöffnung (7) entspricht. Kombinationen diskreter Positionen, bei denen die Blendenelemente (4) diskrete Positionen einnehmen, in denen ihre einander zugewandten Enden (6) aneinander anliegen und keine Durchgangsöffnung (8) begrenzen, so dass die Blendenelemente (4) die emittierte Strahlung komplett abblocken, tragen nicht zum Gesamtenergieverlauf bei und erhalten die Kosten 0. Diese Kombinationen diskreter Positionen finden sich im rechten Diagramm der Figur 5 auf einer sich schräg von links oben nach rechts unten erstreckenden, gestrichelt dargestellten Linie. Für eine dieser Kombinationen verdeutlicht der Pfeil (i) die Korrelation zwischen dem linken und dem rechten Diagramm. Kombinationen diskreter Positionen hingegen, bei denen die Blendenelemente (4) diskrete Positionen einnehmen, in denen sie Durchgangsöffnungen (8) begrenzen, die zum Gesamtenergieverlauf beitragen wenn Röntgenstrahlung durch sie hindurchtritt, werden von den beiden im rechten Diagramm eingezeichneten Dreiecken umschlossen. Diesen Kombinationen innerhalb der Dreiecke werden gemäß der Kostenfunktion die Kosten 1 zugewiesen. Die Pfeile (ii) und (iii) verdeutlichen die Korrelationen zwischen den Energiespitzen im linken Diagramm und den Dreiecken im rechten Diagramm.

Während der Bestrahlung des Patienten (9) bewegt sich die Strahlenquelle (1) entlang der Trajektorie (14) und nimmt dabei sukzessiv Stellungen ein, in denen die Ausstrahlrichtung (5) mit sukzessive aufeinanderfolgenden Einstrahlrichtungen (10, 11, 12, 13) zusammenfällt. Da die Blendenelemente (4) in jeder Stellung der Strahlenquelle (1) Positionen gemäß einer jeweiligen Kombination diskreter Positionen einnehmen, kann eine Folge sukzessiv aufeinanderfolgender Kombinationen diskreter Positionen der beiden Blendenelemente (4) für jeweilige sukzessiv aufeinanderfolgende Einstrahlrichtungen (10, 11, 12, 13) bestimmt werden, mit der die Dosisverteilung bestmöglich erzielt wird, wenn die Strahlenquelle (1) entsprechend dieser Folge in den sukzessiv aufeinanderfolgenden Einstrahlrichtungen (10, 11, 12, 13) Röntgenstrahlung für die vorgegebene Zeitdauer emittiert während die Blendenelemente (4) diskrete Positionen gemäß den jeweils zugehörigen Kombinationen einnehmen.

Die Ermittlung dieser Folge kann mittels eines Suchalgorithmus wie zum Beispiel durch einen "kürzester-Pfad-Algorithmus" bei Berücksichtigung der Beschleunigungen der Blendenelemente (4) erfolgen, welcher aus den zahlreichen Möglichkeiten, Kombinationen diskreter Positionen aufeinanderfolgen zu lassen, in dem Bestreben die vorgegebene Dosisverteilung möglichst zu erreichen, die optimalste bestimmt. Hierbei wird gemäß der vorliegenden Erfindung nicht nur der Beitrag der Kombinationen zu den Gesamtenergieverläufen mittels der Kostenfunktion berücksichtigt, sondern es wird insbesondere auch beachtet, dass infolge der hohen Geschwindigkeit der Strahlenquelle (1) aufeinanderfolgende Kombinationen diskreter Positionen für aufeinanderfolgende Einstrahlrichtungen (10, 11, 12, 13) sehr stark miteinander korreliert sind. So ist es den Blendenelementen (4), die Positionen gemäß einer Kombination diskreter Positionen einnehmen, wenn sich die Strahlenquelle (1) in einer Stellung befindet, in der ihre Ausstrahlrichtung (5) z.B. mit der Einstrahlrichtung (10) zusammenfällt, während der Zeit, in welcher sich die Strahlenquelle (1) in eine nachfolgende Stellung bewegt, in der ihre Ausstrahlrichtung (5) mit der Einstrahlrichtung (11) zusammenfällt, oftmals nicht möglich, in Positionen gemäß aller Kombinationen diskreter Positionen überzugehen, da die Zeit, in welcher sich die Strahlenquelle (1) von der einen Stellung in die andere bewegt, hierfür aufgrund der begrenzten Maximalgeschwindigkeit der Blendenelemente (4) und der von diesen erreichbaren Beschleunigungen zu kurz ist. Dem Gesamtenergieverlauf zwar prinzipiell zuträgliche Kombinationen diskreter Positionen, denen von der Kostenfunktion die Kosten 1 zugewiesen wurden, bleiben für die Blendenelemente (4) deswegen unerreichbar. Im Falle des vorliegenden Steuerverfahrens erfolgt die Ermittlung sukzessiver Kombinationen diskreter Positionen daher in zwei Schritten, die in der Figur 6 anhand eines stark vereinfachten Beispiels erläutert sind.

In der Figur 6 sind für ein Blendenelement (4) diskrete Positionen für jede der Einstrahlrichtungen (10, 11, 12, 13) symbolisch als Kreise dargestellt, die entlang von den Einstrahlrichtungen (10, 11, 12, 13) zugeordneten Linien angeordnet sind. Alle Kreise, die entlang einer Linie angeordnet sind, stehen somit für diskrete Positionen, die von dem Blendenelement (4) eingenommen werden können, wenn sich die Strahlenquelle (1) in einer Stellung befindet, in der ihre Ausstrahlrichtung (5) mit der dieser Linie entsprechenden Einstrahlrichtung (10, 11, 12, 13) zusammenfällt. Wenn die Strahlenquelle (1) beispielsweise eine Stellung einnimmt, in der ihre Ausstrahlrichtung (5) mit der Einstrahlrichtung (10) zusammenfällt, und das Blendenelement (4) die diskrete Position (16) einnimmt, so sind während der Zeit des Bewegens der Strahlenquelle (1) in eine Stellung, in welcher ihre Ausstrahlrichtung (5) mit der Einstrahlrichtung (11) zusammenfällt, aufgrund der beschränkten Maximalgeschwindigkeit des Blendenelementes (4) sowie der begrenzten Beschleunigungen des Blendenelementes (4) in der neuen Stellung der Strahlenquelle (1) nur diskrete Positionen für das Blendenelement (4) erreichbar, die sich zwischen den beiden von der Position (16) ausgehenden gestrichelten Linien befinden. Diese Positionen werden in einem ersten Schritt zusammen mit entsprechenden Positionen des jeweils anderen Blendenelementes (4) des Blendenpaares (3) für mögliche Kombinationen diskreter Positionen für die Einstrahlrichtung (11) vorausgewählt. In einem zweiten Schritt wird von diesen vorausgewählten Kombinationen diejenige Kombination ausgewählt, welcher von der Kostenfunktion die höchsten Kosten zugewiesen werden. In der Figur 6 gehört die diskrete Position (17) zu dieser Kombination. Für den Übergang der Strahlenquelle (1) zur nächstfolgenden Stellung, in der ihre Ausstrahlrichtung (5) mit der Einstrahlrichtung (12) zusammenfällt, werden nun auf analoge Weise wiederum in einem ersten Schritt ausgehend von dieser Kombination diskreter Positionen, welche die Position (17) umfasst, die während der Zeit des Übergangs der Strahlenquelle (1) von dem Blendenelement (4) erreichbaren diskreten Positionen ausgewählt, also in der Figur 6 diejenigen diskreten Positionen für die Einstrahlrichtung (12), die sich zwischen den beiden von der Position (17) ausgehenden gestrichelten Linien befinden, und in einem zweiten Schritt wird aus diesen und den entsprechenden Positionen des jeweiligen anderen Blendenelementes (4) des Blendenpaares (3) diejenige Kombination mit den höchsten Kosten ausgewählt, zu der vorliegend die Position (18) gehört. Auch beim Übergang von der Einstrahlrichtung (12) zur Einstrahlrichtung (13) erfolgt eine Vorauswahl grundsätzlich erreichbarer diskreter Positionen in einem ersten Schritt, bei denen es sich um die zwischen den beiden von der Position (18) ausgehenden gestrichelten Linien befindlichen Positionen handelt. Jedoch finden sich unter diesen vorausgewählten Positionen nun zwei Positionen (19) und (20) die zu Kombinationen diskreter Positionen mit den Kosten 1 gehören, die im Vergleich zu den Kosten 0 der anderen Kombinationen die höchsten sind. Welche dieser Positionen (19) und (20) letztendlich ausgewählt wird, um von dieser dann für den Übergang zurück zur Einstrahlrichtung (10) die nächstfolgende Kombination diskreter Positionen zu bestimmen, wird vom jeweils verwendeten Suchalgorithmus mittels bekannter Verfahren bestimmt. Sofern der Suchalgorithmus zum Beispiel die Kombination mit der Position (20) auswählt, erhält man auf diese Weise für einen Umlauf der Strahlenquelle (1) um den Patienten (9) eine sukzessive Folge von jeweiligen Kombinationen mit diskreten Positionen (16), (17), (18), (20) für die jeweiligen Einstrahlrichtungen (10), (11), (12), (13), und sofern er die Kombination mit der Position (19) auswählt, erhält man eine sukzessive Folge von jeweiligen Kombinationen mit diskreten Positionen (16), (17), (18), (19), die in der Figur 6 beide mittels durchgängiger Linien angedeutet sind. Da zum Erzielen der vorgegebenen Dosisverteilung meistens mehrere Umläufe der Strahlenquelle (1) notwendig sind, kann die auf diese Weise ermittelte Folge für beliebig viele Umläufe beliebig lang sein. Eine derartige Folge von Kombinationen diskreter Positionen wird für alle Blendenelemente (4) jedes Blendenpaares (3) ermittelt.

Schließlich wird im Schritt S6 der Figur 4 die Bestrahlung des Patienten (9) gemäß der im Schritt S5 ermittelten Folgen von Kombinationen diskreter Positionen durchgeführt. Hierzu bewegt sich die Strahlenquelle (1) entlang der Trajektorie (14) und nimmt sukzessiv Stellungen ein, bei denen ihre Ausstrahlrichtung (5) mit jeweiligen der Einstrahlrichtungen (10, 11, 12, 13) übereinstimmt. In diesen Stellungen der Strahlenquelle (1) werden die Blendenelemente (4) aller Blendenpaare (3) entsprechend den jeweiligen Kombinationen diskreter Positionen für diese Stellung positioniert, und das Strahlenerzeugungsmittel (2) emittiert Röntgenstrahlung für die vorgegebene Zeitdauer. Dies geschieht so lange, bis die Folgen von Kombinationen diskreter Positionen für alle Blendenpaare (3) durchlaufen sind. Das Verfahren endet daraufhin mit dem Schritt S7.

Während der Schritt S6, bei dem die eigentliche Bestrahlung des Patienten (9) stattfindet, von der Bestrahlungseinrichtung durchgeführt wird deren Teil die Strahlungsquelle (1) ist, werden die Schritte S3-S5 für gewöhnlich nicht von der Bestrahlungseinrichtung sondern von einer Datenverarbeitungseinrichtung ausgeführt. Nachdem die Datenverarbeitungseinrichtung die Folgen von Kombinationen diskreter Positionen für die Blendenelemente (4) ermittelt hat, werden sie der Bestrahlungseinrichtung übergeben, damit diese die Bestrahlung entsprechend der Folgen durchführen kann.

### Bezugszeichenliste

- 1.: Strahlenquelle
- 2.: Strahlenerzeugungsmittel
- 3.: Blendenpaar
- 4.: Blendenelement
- 5.: Ausstrahlrichtung
- 6.: Enden
- 7.: Durchgangsmaximalöffnung
- 8.: Durchgangsöffnung
- 9.: Objekt
- 10.: Einstrahlrichtung
- 11.: Einstrahlrichtung
- 12.: Einstrahlrichtung
- 13.: Einstrahlrichtung
- 14.: Trajektorie
- 15.: Volumenelement
- 16.: Kombination diskreter Positionen
- 17.: Kombination diskreter Positionen
- 18.: Kombination diskreter Positionen
- 19.: Kombination diskreter Positionen
- 20.: Kombination diskreter Positionen

## Patentansprüche

1. Bestrahlungseinrichtung zum Bestrahlen eines Objektes (9) aus wenigstens zwei relativ zum Objekt (9) unterschiedlichen Einstrahlrichtungen (10, 11, 12, 13), wobei die Bestrahlungseinrichtung wenigstens eine Strahlenquelle (1) zum Emittieren einer Strahlung mit einem vorgegebenen Intensitätsprofil in einer Ausstrahlrichtung (5) relativ zur Strahlenquelle (1) aufweist, wobei die Strahlenquelle (1) zwischen wenigstens zwei jeweiligen Stellungen bewegbar ist, in denen ihre Ausstrahlrichtung (5) mit einer jeweiligen der Einstrahlrichtungen (10, 11, 12, 13) zusammenfällt, und wobei die Strahlenquelle (1) wenigstens ein Blendenpaar (3) mit zwei für die emittierte Strahlung undurchlässigen und entlang einer zur Ausstrahlrichtung gewinkelten Geraden mit einer Maximalgeschwindigkeit verschiebbaren Blendenelementen (4) mit einander zugewandten Enden (6) umfasst, die zum Einnehmen jeweiliger Positionen eingerichtet sind, in denen die Enden (6) einen größtmöglichen Abstand voneinander aufweisen und einander gegenüberliegende Begrenzungen einer Durchgangsmaximalöffnung (7) größtmöglicher Breite für wenigstens einen Teil der emittierten Strahlung bilden, und die ferner zum Einnehmen einer Mehrzahl weiterer Positionen eingerichtet sind, in denen die Enden (6) einen kleineren als den größtmöglichen Abstand voneinander aufweisen, wobei sie entweder einander gegenüberliegende Begrenzungen jeweiliger Durchgangsöffnungen (8) bilden, die Teilabschnitte der Durchgangsmaximalöffnung (7) sind, oder aneinander anliegen, wobei die Bestrahlungseinrichtung ferner einen Computer aufweist, der eingerichtet ist, die folgenden Schritte durchzuführen:
a) Vorgeben einer zu erzielenden Dosisverteilung, welche eine jeweiligen Volumenelementen (15) des Objektes (9) von der Strahlung zuzuführende Energie beschreibt;
b) Festlegen sukzessiv aufeinanderfolgender Einstrahlrichtungen (10, 11, 12, 13), aus denen das Objekt (9) zu bestrahlen ist;
c) Ableiten von jeweiligen Gesamtenergieverläufen für jede Einstrahlrichtung (10, 11, 12, 13) aus der Dosisverteilung und dem Intensitätsprofil der emittierten Strahlung, wobei ein Gesamtenergieverlauf einer gegebenen Einstrahlrichtung (10, 11, 12, 13) für Punkte in der Durchgangsmaximalöffnung (7) die Gesamtenergie angibt, welche von der Strahlung durch diese Punkte zu transportieren ist, damit nach erfolgter Bestrahlung des Objektes (9) aus allen Einstrahlrichtungen (10, 11, 12, 13) entsprechend den jeweiligen Gesamtenergieverläufen die Dosisverteilung erzielt wird;
d) Erstellen von Kostenfunktionen für jede Einstrahlrichtung (10, 11, 12, 13) auf Grundlage der Gesamtenergieverläufe, welche Kombinationen (16, 17, 18, 19, 20) diskreter Positionen der beiden Blendenelemente (4) jeweilige Kosten entsprechend eines Beitrages derjenigen Energie zum jeweiligen Gesamtenergieverlauf zuweisen, die von Strahlung mit dem vorgegebenen Intensitätsprofil in einer vorgegebenen Zeitdauer durch diejenige Durchgangsöffnung (8) transportiert wird, die von den Blendenelementen (4) bei Einnahme von diskreten Positionen entsprechend der jeweiligen Kombination (16, 17, 18, 19, 20) diskreter Positionen begrenzt wird;
e) Ermitteln einer Folge sukzessiv aufeinanderfolgender Kombinationen (16, 17, 18, 19, 20) diskreter Positionen der beiden Blendenelemente (4) für jeweilige sukzessiv aufeinanderfolgende Einstrahlrichtungen (10, 11, 12, 13) unter Einbeziehung der Kostenfunktion, mit der die Dosisverteilung bestmöglich erzielt wird, wenn die Strahlenquelle (1) entsprechend dieser Folge in den sukzessiv aufeinanderfolgenden Einstrahlrichtungen (10, 11, 12, 13) Strahlung für die vorgegebene Zeitdauer emittiert während die Blendenelemente (4) diskrete Positionen gemäß den jeweiligen Kombinationen (16, 17, 18, 19, 20) diskreter Positionen der Folge einnehmen, wobei für Kombinationen (16, 17, 18, 19, 20) diskreter Positionen der Folge jeweilige nachfolgende Kombinationen (16, 17, 18, 19, 20) diskreter Positionen der Folge ermittelt werden, indem ausgehend von einer Kombination (16, 17, 18, 19, 20) diskreter Positionen für eine jeweilige Einstrahlrichtung (10, 11, 12, 13) als mögliche Kombinationen (16, 17, 18, 19, 20) diskreter Positionen einer jeweils nachfolgenden Einstrahlrichtung (10, 11, 12, 13) diejenigen Kombinationen (16, 17, 18, 19, 20) diskreter Positionen bestimmt werden, welche von den Blendenelementen (4) mit der Maximalgeschwindigkeit in derjenigen Zeit erreichbar sind, in welcher sich die Strahlenquelle (1) von einer Stellung, in welcher deren Ausstrahlrichtung (5) mit der jeweiligen Einstrahlrichtung (10, 11, 12, 13) zusammenfällt zu einer Stellung bewegt, in welcher deren Ausstrahlrichtung (5) mit der nachfolgenden Einstrahlrichtung (10, 11, 12, 13) zusammenfällt;
f) sukzessives Bewegen der Strahlenquelle (1) in Stellungen sowie Positionieren der Blendenelemente (4) gemäß jeweiliger Kombinationen (16, 17, 18, 19, 20) diskreter Positionen entsprechend der Folge, wobei die Strahlenquelle (1) in jeder Stellung die Strahlung mit dem vorgegebenen Intensitätsprofil für die vorgegebene Zeitdauer emittiert.

2. Bestrahlungseinrichtung nach Anspruch 1, die eine Mehrzahl von interdigitierend angeordneten Blendenpaaren (3) aufweist.

3. Bestrahlungseinrichtung nach einem der Ansprüche 1 oder 2, die dazu eingerichtet ist, im Schritt e) für Kombinationen (16, 17, 18, 19, 20) diskreter Positionen der Folge jeweilige nachfolgende Kombinationen (16, 17, 18, 19, 20) diskreter Positionen der Folge unter zusätzlicher Berücksichtigung von Beschleunigungen der Blendenelemente (4) zu ermitteln.

4. Bestrahlungseinrichtung nach einem der vorhergehenden Ansprüche, die dazu eingerichtet ist, im Schritt e) die nachfolgenden Kombinationen (16, 17, 18, 19, 20) diskreter Positionen der Folge unter Berücksichtigung der Maximalgeschwindigkeit der Blendenelemente (4) in einem ersten Schritt vorauszuwählen und in einem zweiten Schritt von den im ersten Schritt vorausgewählten Kombinationen (16, 17, 18, 19, 20) diskreter Positionen wenigstens eine Kombination (16, 17, 18, 19, 20) diskreter Positionen in Abhängigkeit von den ihr zugewiesenen Kosten auszuwählen.

5. Bestrahlungseinrichtung nach einem der vorhergehenden Ansprüche, die eine Strahlenquelle (1) mit einer Mehrzahl von Blendenpaaren (3) aufweist und die dazu eingerichtet ist, die Schritte c) bis f) für jedes der Blendenpaare (3) durchzuführen.

6. Bestrahlungseinrichtung nach einem der vorhergehenden Ansprüche, die dazu eingerichtet ist, wenigstens einen der Schritte c) bis e) von einer Datenverarbeitungsvorrichtung auszuführen.

7. Bestrahlungseinrichtung nach einem der vorhergehenden Ansprüche, die dazu eingerichtet ist, den Schritt a) wenigstens zweimal oder mehrfach auszuführen oder die Schritte a) bis e) wenigstens zweimal oder mehrfach auszuführen.

8. Bestrahlungseinrichtung nach einem der vorhergehenden Ansprüche, die dazu eingerichtet ist, während des Bewegens von wenigstens einer der Stellungen zur jeweiligen nächstfolgenden Stellung kontinuierlich Strahlung von der Strahlenquelle (1) zu emittieren und/oder die Strahlenquelle (1) während des Bewegens von wenigstens einer der Stellungen zur jeweiligen nächstfolgenden Stellung zu deaktivieren.

9. Computerprogrammprodukt umfassend Befehle, die bei der Ausführung des Programms durch den Computer der Bestrahlungsvorrichtung gemäß einem der vorherigen Ansprüche, diesen veranlassen, die Schritte a) - f) gemäß einem der vorherigen Ansprüche ausführen.

10. Speichermedium, auf dem ein Computerprogrammprodukt nach Anspruch 9 gespeichert ist.

## Claims

1. An irradiation device for irradiating an object (9) from at least two different directions of radiation incidence (10, 11, 12, 13) relative to the object (9), wherein the irradiation device has at least one radiation source (1) for emitting radiation with a pre-determined intensity profile in a radiation emitting direction (5) relative to the radiation source (1), wherein the radiation source (1) is movable between at least two respective positions, in which its radiation emitting direction (5) coincides with a respective one of the directions of radiation incidence (10, 11, 12, 13), and wherein the radiation source (1) comprises at least one pair of apertures (3) with two aperture elements (4) with ends (6) facing each other, which are impermeable for the emitted radiation and displaceable along a straight line angled to the radiation emitting direction with maximum speed, which ends are arranged for the assumption of respective positions, in which the ends (6) have a maximum possible distance from each other and form boundaries opposite each other of a maximum passage opening (7) of maximum possible width for at least a part of the emitted radiation, and which furthermore are arranged for the assumption of a plurality of further positions, in which the ends (6) have a less than the maximum possible distance from each other, wherein they either form boundaries opposite each other of respective passage openings (8), which are sub-sections of the maximum passage opening (7), or abut against each other, wherein the irradiation device furthermore has a computer, which is arranged, to carry out the following steps:
a) Specifying a dose distribution to be achieved, which describes an energy to be supplied to respective volume elements (15) of the object (9) by the radiation;
b) successively defining successive directions of radiation incidence (10, 11, 12, 13), from which the object (9) is to be irradiated;
c) deriving respective total energy flows for each direction of radiation incidence (10, 11, 12, 13) from the dose distribution and the intensity profile of the emitted radiation, wherein a total energy flow of a given direction of radiation incidence (10, 11, 12, 13) indicates the total energy for points in the maximum passage opening (7), which is to be transported by the radiation through these points, so that after the irradiation of the object (9) from all directions of radiation incidence (10, 11, 12, 13) the dose distribution is achieved corresponding to the respective total energy flows;
d) creating cost functions for each direction of radiation incidence (10, 11, 12, 13) on the basis of the total energy flows, which assign respective costs to combinations (16, 17, 18, 19, 20) of discrete positions of both aperture elements (4) corresponding to a contribution of that energy to the respective total energy flow, which is transported by radiation with the predetermined intensity profile in a predetermined time period through that passage opening (8), which is bounded by the aperture elements (4) assuming discrete positions corresponding to the respective combination (16, 17, 18, 19, 20) of discrete positions;
e) determining a sequence successively of successive combinations (16, 17, 18, 19, 20) of discrete positions of both aperture elements (4) for respective successive directions of radiation incidence (10, 11, 12, 13) including the cost function, with which the dose distribution is achieved as best as possible, if the radiation source (1) corresponding to this sequence emits radiation in the successive directions of radiation incidence (10, 11, 12, 13) for the pre-determined time period while the aperture elements (4) assume discrete positions according to the respective combinations (16, 17, 18, 19, 20) of discrete positions of the sequence, wherein for combinations (16, 17, 18, 19, 20) of discrete positions of the sequence respective subsequent combinations (16, 17, 18, 19, 20) of discrete positions of the sequence are determined, while proceeding from a combination (16, 17, 18, 19, 20) of discrete positions for a respective direction of radiation incidence (10, 11, 12, 13) as possible combinations (16, 17, 18, 19, 20) of discrete positions of in each case a subsequent direction of radiation incidence (10, 11, 12, 13) those combinations (16, 17, 18 , 19, 20) of discrete positions are determined, which can be achieved by the aperture element (4) with the maximum speed in that time, in which the radiation source (1) of a position in which its radiation emitting direction (5) coincides with the respective direction of radiation incidence (10, 11, 12, 13), moves to a position, in which its radiation emitting direction (5) coincides with the subsequent direction of radiation incidence (10, 11, 12, 13);
f) successive movement of the radiation source (1) into positions as well as positioning of aperture elements (4) according to respective combinations (16, 17, 18, 19, 20) of discrete positions corresponding to the sequence, wherein the radiation source (1) in each position emits the radiation with the pre-determined intensity profile for the pre-determined time period.

2. An irradiation device according to Claim 1, which has a plurality of interdigitatingly arranged pairs of apertures (3).

3. An irradiation device according to one of Claims 1 or 2, which is arranged in the step e) to determine for combinations (16, 17, 18, 19, 20) of discrete positions of the sequence respective subsequent combinations (16, 17, 18, 19, 20) of discrete positions of the sequence additionally taking into account accelerations of the aperture elements (4).

4. An irradiation device according to any of the preceding claims, which is arranged in step e) to pre-select the subsequent combinations (16, 17, 18, 19, 20) of discrete positions of the sequence taking into account the maximum speed of the aperture elements (4) in a first step and in a second step to select from the combinations (16, 17, 18, 19, 20) of discrete positions pre-selected in the first step at least one combination (16, 17, 18, 19, 20) of discrete positions depending on the costs assigned to it.

5. An irradiation device according to any one of the preceding claims, which has a radiation source (1) with a plurality of pairs of apertures (3) and is arranged to carry out the steps c) to f) for each of the pairs of apertures (3).

6. An irradiation device according to any one of the preceding claims, which is arranged to carry out at least the steps c) to e) by a data processing device.

7. An irradiation device according to any one of the preceding claims, which is arranged to carry out the step a) at least twice or repeatedly or to carry out the steps a) to e) at least twice or repeatedly.

8. An irradiation device according to any one of the preceding claims, which is arranged to continuously emit the radiation from the radiation source (1) during the movement from at least one of the positions to the respective next following position and/or to deactivate the radiation source (1) during the movement from at least one of the positions to the respective next following position.

9. A computer program product comprising commands, which in the execution of the program by the computer of the irradiation device according to one of the preceding claims, causes the latter to carry out the steps a) to f) according to one of the preceding claims.

10. A storage medium on which a computer program product according to Claim 9 is stored.

## Revendications

1. Dispositif d'irradiation pour l'irradiation d'un objet (9) à partir d'au moins deux directions d'incidence (10, 11, 12, 13) différentes par rapport à l'objet (9), le dispositif d'irradiation comprenant au moins une source de rayonnement (1) pour l'émission d'un rayonnement avec un profil d'intensité prédéterminé dans une direction d'émission (5) par rapport à la source de rayonnement (1), la source de rayonnement (1) étant mobile entre au moins deux positions au niveau desquelles sa direction d'émission (5) coïncide avec une des directions d'incidence (10, 11, 12, 13) et la source de rayonnement (1) comprenant au moins une paire de diaphragmes (3) avec deux éléments de diaphragmes (4), imperméables au rayonnement émis et pouvant être déplacés, avec une vitesse maximale, le long d'une droite formant un angle avec la direction d'émission, avec des extrémités (6) orientées l'une vers l'autre, qui sont conçues pour adopter des positions au niveau desquelles les extrémités (6) présentent la distance la plus importante possible entre elles et formant des limitations, opposées entre elles, d'une ouverture maximale de passage (7) de la largeur la plus importante possible pour au moins une partie du rayonnement émis et qui sont en outre conçues pour adopter une pluralité d'autres positions au niveau desquelles les extrémités (6) présentent entre elles une distance inférieure à la distance la plus importante possible, celle-ci formant soit des limitations opposées entre elles d'ouvertures de passage (8) correspondantes, qui sont des portions de l'ouverture maximale de passage (7), ou s'appuyant l'une contre l'autre, le dispositif d'irradiation comprenant en outre un ordinateur qui est conçu pour exécuter les étapes suivantes :
a) prédétermination d'une répartition de dose à obtenir, qui définit une énergie que le rayonnement doit apporter à des éléments de volume (15) de l'objet (9) ;
b) la détermination de directions d'incidences (10, 11, 12, 13) successives, à partir desquelles l'objet (9) doit être irradié ;
c) déduction de courbes d'énergie totale pour chaque direction d'incidence (10, 11, 12, 13) à partir de la répartition de dose et du profil d'intensité du rayonnement émis, une courbe d'énergie totale d'une direction d'incidence (10, 11, 12, 13) donnée indiquant, pour certains points dans l'ouverture maximale de passage (7), l'énergie totale qui doit être transportée par le rayonnement à travers ces points, afin que, après l'irradiation de l'objet (9) dans toutes les directions d'incidences (10, 11, 12, 13), la répartition de dose soit obtenue en fonction des courbes d'énergie totale ;
d) création de fonctions de coûts pour chaque direction d'incidence (10, 11, 12, 13) sur la base des courbes d'énergie totale, qui attribuent à des combinaisons (16, 17, 18, 19, 20) de positions discrètes des deux éléments de diaphragmes (4) des coûts correspondant à une contribution de l'énergie respective à la courbe d'énergie totale, qui est transportée par le rayonnement avec le profil d'intensité pendant une période prédéterminée à travers l'ouverture de passage (8) qui est limitée par les éléments de diaphragmes (4) lors de l'adoption de positions discrètes correspondant à la combinaison respective (16, 17, 18, 19, 20) de positions discrètes ;
e) détermination d'une suite de combinaisons successives (16, 17, 18, 19, 20) de positions discrètes des deux éléments de diaphragmes (4) pour des directions d'incidence successives (10, 11, 12, 13) à partir de la fonction de coût, avec laquelle la meilleure répartition de dose possible est obtenue, lorsque la source de rayonnement (1) émet, selon cette suite dans les directions d'incidence successives (10, 11, 12, 13), un rayonnement pendant la période prédéterminée, tandis que les éléments de diaphragmes (4) adoptent des positions discrètes selon les combinaisons respectives (16, 17, 18, 19, 20) de la suite, des positions discrètes de la suite de combinaisons suivantes (16, 17, 18, 19, 20) de positions discrètes de la suite étant déterminées pour des combinaisons (16, 17, 18, 19, 20) de positions discrètes de la suite, grâce au fait que, à partir d'une combinaison (16, 17, 18, 19, 20) de positions discrètes pour une direction d'incidence respective (10, 11, 12, 13), en tant que combinaisons possibles (16, 17, 18, 19, 20) de positions discrètes d'une direction d'incidence suivante (10, 11, 12, 13), les combinaisons (16, 17, 18, 19, 20) de positions discrètes déterminées sont celles qui sont accessibles par les éléments de diaphragmes (4) avec la vitesse maximale dans le temps pendant lequel la source de rayonnement (1) se déplace d'une position, au niveau de laquelle sa direction d'émission (5) coïncide avec la direction d'incidence respective (10, 11, 12, 13) vers une position au niveau de laquelle sa direction d'émission (5) coïncide avec la direction d'incidence suivante (10, 11, 12, 13) ;
f) déplacement successif de la source de rayonnement (1) vers des positions ainsi que positionnement des éléments de diaphragmes (4) selon des combinaisons respectives (16, 17, 18, 19, 20) de positions discrètes selon la suite, la source de rayonnement (1) émettant, dans chaque position, le rayonnement avec le profil d'intensité prédéterminé pendant la période prédéterminée.

2. Dispositif d'irradiation selon la revendication 1, qui comprend une pluralité de paires de diaphragmes (3) disposés de manière entrecroisée.

3. Dispositif d'irradiation selon l'une des revendications 1 ou 2, qui est conçu pour déterminer, dans l'étape e), pour des combinaisons (16, 17, 18, 19, 20) de positions discrètes de la suite de combinaisons suivantes (16, 17, 18, 19, 20) de positions discrètes de la suite en tenant en outre compte des accélérations des éléments de diaphragmes (4).

4. Dispositif d'irradiation selon l'une des revendications précédentes, qui est conçu pour pré-sélectionner, dans l'étape e), les combinaisons suivantes (16, 17, 18, 19, 20) de positions discrètes de la suite en tenant compte de la vitesse maximale des éléments de diaphragmes (4) dans une première étape et pour sélectionner, dans une deuxième étape, à partir des combinaisons (16, 17, 18, 19, 20) de positions discrètes présélectionnées dans la première étape, au moins une combinaison (16, 17, 18, 19, 20) de positions discrètes en fonction des coûts qui lui sont attribués.

5. Dispositif d'irradiation selon l'une des revendications précédentes, qui comprend une source de rayonnement (1) avec une pluralité de paires de diaphragmes (3) et qui est conçu pour exécuter les étapes c) à f) pour chacune des paires de diaphragmes (3).

6. Dispositif d'irradiation selon l'une des revendications précédentes, qui est conçu pour exécuter au moins une des étapes c) à e) à l'aide d'un dispositif de traitement de données.

7. Dispositif d'irradiation selon l'une des revendications précédentes, qui est conçu pour exécuter l'étape a) au moins deux fois ou plusieurs fois ou les étapes a) à e) au moins deux fois ou plusieurs fois.

8. Dispositif d'irradiation selon l'une des revendications précédentes, qui est conçu pour émettre, pendant le déplacement d'au moins une des positions vers la position suivante, un rayonnement continu à l'aide de la source de rayonnement (1) et/ou pour désactiver la source de rayonnement (1) pendant le déplacement d'au moins une des positons vers la position suivante.

9. Produit de programme informatique comprenant des instructions qui, lors de l'exécution du programme par l'ordinateur du dispositif d'irradiation selon l'une des revendications précédentes, font en sorte que celui-ci exécute les étapes a) à f) selon l'une des revendications précédentes.

10. Support d'enregistrement sur lequel un produit de programme informatique selon la revendication 9 est enregistré.
